# EUROPEAN PATENT APPLICATION

(11) **EP 4 389 166 A1**
(43) Date of publication of application: **26.06.2024**
(21) Application number: 22215586.3
(22) Date of filing: 21.12.2022
(51) Int. Cl.: A61M 1/06

(54) **BREAST PUMP**

(71) Applicant: Medela Holding AG, 6340 Baar (CH)
(72) Inventor: HOENER, Sebastian, 8800 Thalwil (CH); THÜRING, Martin, 5634 Merenschwand (CH)
(74) Representative: Grünecker Patent- und Rechtsanwälte PartG mbB

(57) **Abstract**

The present invention relates to a breast pump (1) shaped at least in part to fit inside a bra and built to accumulate milk (M) expressed from a breast of a lactating mother in a milk container (36), the breast pump (1) comprising
- a housing (2),
- a suction source (4),
- a breast shield element (24) with a nipple tunnel (28) adapted to receive a nipple,
- a sensor means (210) including an emitter (211) for an emission of electromagnetic waves and a receiver (212) for a reception of the electromagnetic waves, designed to provide a sensor signal for a detection of the milk (M),
- a controller (200) for controlling the suction source (4) and adapted to receive the sensor signal, and
- milk (M),
wherein the transparent section (360) has a protrusion (362) arranged to be in front of and to face away from the corresponding sensor means (210) in order to be wetted by the milk (M) and in order to reflect and/or refract the electromagnetic waves from the emitter (211).

## Description

The present invention relates to a breast pump shaped at least in part to fit inside a bra. The breast pump comprises a housing, a suction source, a breast shield element, a sensor means, a controller and a milk container. The sensor means is designed to provide a sensor signal for a detection of milk. The milk container is adapted to contain a predetermined amount of a/the milk. The breast pump is meant for expressing milk of a breast-feeding and/or lactating mother and to accumulate the milk in the container.

Such breast pump is e.g. known from WO 2022/038006 A1. In this known breast pump the sensor means is used to detect a first milk when it reaches the bottom of the milk container. However, the sensor means is insufficient for an efficient milk expression in that it cannot quantify the actual amount/volume of milk in the milk container.

The breast pump of WO 2018/229504 A1 may quantify the amount of milk in the milk container due to its sensor means being placed above a surface of the milk in the milk container measuring a distance to the surface that depends on the fill level of the milk container. The measurement is however adversely affected by the electromagnetic waves travelling through the gas volume above the milk, which may contain milk vapor, air and contamination.

It is an object of the present invention to provide a breast pump which provides a better detection of milk and/or quantification of the actual amount of milk in the milk container.

As a solution to this problem, it is suggested to provide a breast pump shaped at least in part to fit inside a bra and built to accumulate milk expressed from a breast of a lactating mother in a milk container, the breast pump comprising
- a housing,
- a suction source,
- a breast shield element with a nipple tunnel adapted to receive a nipple,
- a sensor means including an emitter for an emission of electromagnetic waves and a receiver for a reception of the electromagnetic waves, designed to provide a sensor signal for a detection of the milk,
- a controller for controlling the suction source and adapted to receive the sensor signal, and
- a milk container which is adapted to contain a predetermined amount of the milk and including a transparent section which is at least partially transparent to the electromagnetic waves and arranged to be in contact with the milk.

According to the invention it is suggested that the transparent section has a protrusion arranged to be in front of and to face away from the corresponding sensor means in order to be wetted by the milk and in order to reflect and/or refract the electromagnetic waves from the emitter.

The breast pump may be shaped at least in part to fit inside the bra. Particularly, the breast pump may be shaped at least in part to be wearable inside a bra. In other words, the breast pump may be put inside a worn bra being supported by the bra.

Due to the invention, there is a possibility for a redundant and/or more precise and accurate quantification, i.e. measurement and/or estimation, of the actual amount of milk in the milk container, especially at the first fill level conducted through at least two transparent sections each directly facing the milk on one side and/or by means of the protrusion in the transparent section(s).

The first fill level may pertain to between 100 ml and 120 ml of milk with the milk container in an upright position. The lowermost level pertains to an amount of essentially zero in the milk container. In other words, the first fill level shall be above the bottom of the milk container. The first fill level not being the lowermost fill level not only may enable a detection of first milk, but also a detection/estimation of an actual amount of milk expressed in order to give feedback to the mother. It may be possible to detect if the milk container is full of milk to stop the suction source.

The transparent section may have the protrusion. The protrusion is e.g. a cone and/or an edge that protrudes in the inside of the milk container. This means that the protrusion may point towards the milk but as well may be surrounded by the milk. By means of the protrusion the sensor means may provide a signal that enables a more accurate detection of the presence of milk. The protrusion makes it possible to locate the surface of the milk more precise in the vicinity of the sensor means, especially since the cone has a very small height that is affected by the milk being present or absent.

The protrusion may be shaped such that the emitted waves are reflected back to the receiver when the cone is in contact with ambient air, but are refracted and/or reflected in a direction away from the receiver when the protrusion is in contact with the milk. As such, the protrusion may enable that the electromagnetic waves can be reflected to the sensor means in case the milk container is empty, which as well provides a possibility to check a correct attachment of the milk container within the breast pump.

The sensor means may be using less power for the generation of the sensor signal. Especially when two sensor means are adopted at the first fill level together with the protrusion, the measurement can be most accurate and precise.

The invention has a variety of other benefits that may even be enhanced in preferred embodiments mentioned later. The currently known benefits are listed as follows.

There is a more accurate measurement of milk amount and a more precise amount tracking possible due to the invention.

In addition, the invention may provide the possibility to detect if the breast pump is correctly assembled or closed.

The emitter may be an infrared emitter diode and/or the receiver an infrared receiver diode. Infrared waves are not visible to the human eye and health-wise acceptable.

The emitter and/or the receiver may point towards an emission and/or reception direction. The emitter and the receiver may point towards the same direction and/or the same position. Thus, the waves can be present rather concentrated and not spread.

The emitter and the receiver may be attached to each other and/or placed next to each other and/or may face towards, at least essentially, the same direction and/or be arranged in order to have the emitted electromagnetic waves be reflected at the transparent section and detected by the receiver. The sensor means may face at least essentially in parallel to the surface of the milk in the milk container particularly when the milk container is in an upright position. The sensor means may be provided as an assembly of emitter and receiver, and especially a crosstalk inhibitor between emitter and receiver. The crosstalk inhibitor absorbs and/or reflects waves. The sensor means may be present in the form of emitter and receiver mounted on a printed circuit board next to each other, in particular with the crosstalk inhibitor between them, in particular pointing towards the same direction, e.g. towards the transparent section.

The two or more of the sensor means may be distributed, at least essentially, especially pairwise, across two or more fill levels above the lowermost level. There may be three, four or more fill levels provided, where each fill level may comprise and/or correspond to at least one of the sensor means. As such, there are more fill levels for a more precise milk amount measurement and a better plausibility check. In fact, in case of at least two fill levels each with at least two sensor means, there may be a tilt detection possible from the milk level only contacting distinct transparent sections at distinct sensor means.

The first fill level may be the highest fill level. The difference between fill levels may amount e.g. 10, 20, 25 or 30 ml. The fill levels may be placed at at least two of 20, 50, 80 and 120 ml +/- 5 or 10 ml of milk amount in the milk container in an upright position thereof.

An opening of the housing for the nipple tunnel may be arranged below the first fill level, and/or above the first fill level, and/or above or below at least one of the fill levels, and/or between two of the sensor means. As the opening may serve for the transportation of milk from the nipple tunnel to the milk container, and as the nipple in the nipple tunnel may also come close to the fill level(s) and the sensor means, a distinct placement is beneficial to a first milk detection, to a detection of nipple/areola placement and to a nipple latching and engagement detection.

A spout of the milk container adapted to pour milk out of the milk container and/or
a venting hole of the milk container for a venting of the milk container may be arranged above the first fill level and/or between two of the sensor means. In this regard, an easy pouring of milk without a complicated disassembly and/or an automatic ventilation of the milk container may be possible.

The transparent section may be integrated in the milk container, especially monolithically. As such, a corresponding part of the milk container, such as a lid element, may be produced integrally together with the transparent section(s). The transparent section may be a flat section of the milk container abutting the housing, preferably part of a/the lid element of the milk container. This is beneficial to a minimal size of the transparent sections reducing the cost and a fluid tightness in the area thereof.

The transparent section may be harder and/or more transparent to the electromagnetic waves relative to a different section of the milk container. The transparent section may be made of plastics, in particular amorphous plastics. This helps for scratch resistance when the milk container is handled separate from the sensor means and/or the housing with the sensor means.

A light guidance element, such as a lens and/or a light pipe, might be provided. At least one or all of the sensor means may be in contact with a/the light guidance element, such as a lens and/or a light pipe. As such, the sensor means may be protected somewhat by the light guidance element which may be a plastic or glass part. The light guidance element primarily serves to direct the waves in the sense of an optical lens. The light guidance element may be integrated in the housing, especially in front of an opening of the housing, in particular from the inside of the housing. The light guidance element may as well be integrated in the milk container, especially protruding therefrom towards the sensor means and/or the housing. As such, the light guidance element may be produced by injection molding, especially injection molding of the housing and/or the milk container, in particular the lid element and/or the transparent section. By means of the light guidance element ambient noise waves may be reduced.

It might be possible that there are two or more light guidance elements and/or the light guidance element is implemented on the receiver and/or emitter side, especially within the receiver and/or the emitter.

A crosstalk inhibitor may be provided. The light guidance element may be divided by a/the crosstalk inhibitor. The crosstalk inhibitor particularly inhibits that the emitted electromagnetic waves are reflected and/or transported within the light guidance element towards the receiver. In other words, using the crosstalk inhibitor particularly provokes that the received electromagnetic waves from the emitter must have left the light guidance element at least once. This supports an accurate measurement.

The crosstalk inhibitor may be placed dividing two or more of the light guidance element and the emitter and the receiver. The crosstalk inhibitor may have more than one part. The crosstalk inhibitor may be supported by - especially directly or indirectly mechanically connected to - the controller, especially a/the printed circuit board having the corresponding sensor means, and/or may be supported by the housing and/or the light guidance element.

An encapsulation element may be provided. The light guidance element may be covered by an/the encapsulation element. As such, the light guidance element may be protected by a separate part transparent to the electromagnetic waves, a masking element, a film, a foil, a lacquer and/or a surface layer. While the light guidance element may be used to primarily direct the electromagnetic waves, the encapsulation element may be used to primarily protect the light guidance element but merely to have the waves be transmitted by said encapsulation element only. The encapsulation element may be provided on the side facing the sensor means and/or on the side facing the transparent section.

The encapsulation element may be produced by injection molding, especially injection molding of the light guidance element, the housing and/or the milk container, in particular the lid element and/or the transparent section.

On the side opposite the sensor means, the light guidance element and/or the encapsulation element may be arranged at a distance to the transparent section of no more than 1 mm in order to have a good sensor signal corresponding to the presence of milk. The light guidance element or the encapsulation element may as well be in contact with the transparent section for a most accurate measurement.

The transparent section and/or the protrusion may comprise at least one of a prism, a nose, a cone, a V-shape, a roof-shape, a spherical segment and a pyramid. In particular, the protrusion may consist of or comprise such a shape. This helps so that the sensor means receives back the emitted waves when the milk is not present.

The housing, the suction source, the breast shield element, the sensor means and the milk container may be shaped to be joined for forming a breast pump adapted to fit inside the bra. The controller, the housing and the suction source may as well be joined therewith. As such, a very compact breast pump may be provided, where even the milk is stored in the vicinity of the breast and not outside. This provides mobility to the mother, keeps the milk warm and reduced energy consumption of the suction source.

The detection/estimation of the actual amount of milk in the milk container with the help of the sensor means according to the invention even enables to detect if the milk container and/or the lid element is/are correctly seated in their end positions. This is crucial to the success of the pumping since the breast pump will not seal properly otherwise and pumping success might be compromised.

The mother may be automatically informed by an alarm e.g. on a smartphone if the amount in the milk container reaches a critical level, so that the suction source may be stopped. This is especially important for user happiness in in-bra pumping use.
Fig. 1 is an exploded view of the individual components and elements of a breast pump having a milk container having transparent sections;
Fig. 2 is a sectional view of the breast pump in an assembled state;
Fig. 3 is an enlarged detail of Fig. 2;
Fig. 4 is a rear view of the breast pump;
Fig. 5 is a front view of the breast pump;
Fig. 6 is a top view of the breast pump;
Fig. 7 is a modified sectional view of the breast pump having a controller and sensor means; and
Fig. 8A-B shows the effect of the presence of milk on the path of electromagnetic waves of a sensor means in another second embodiment.

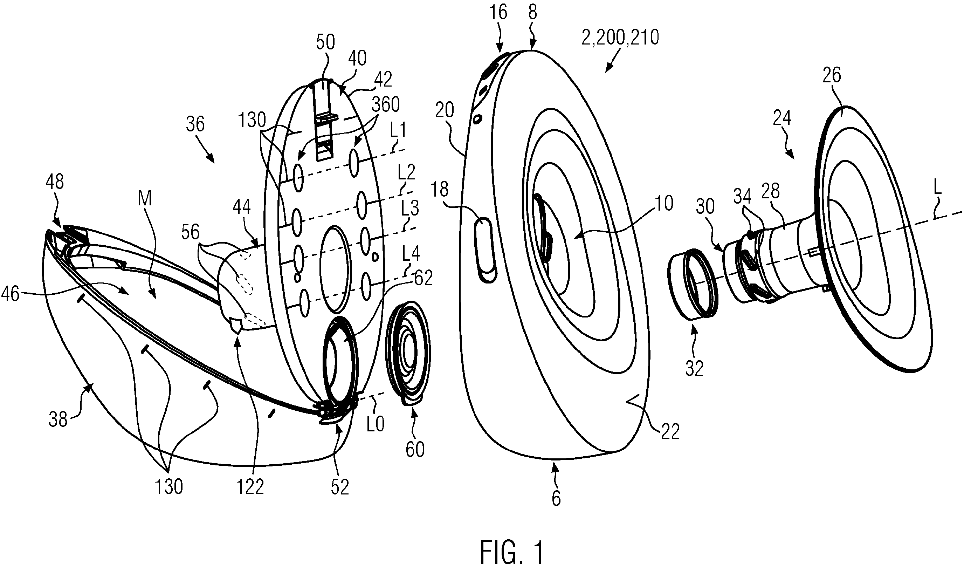

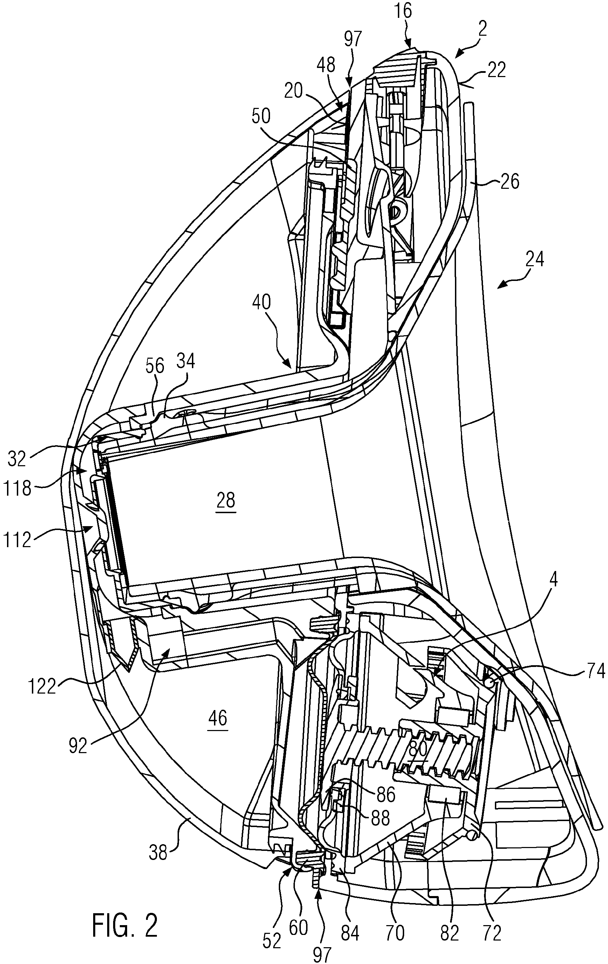

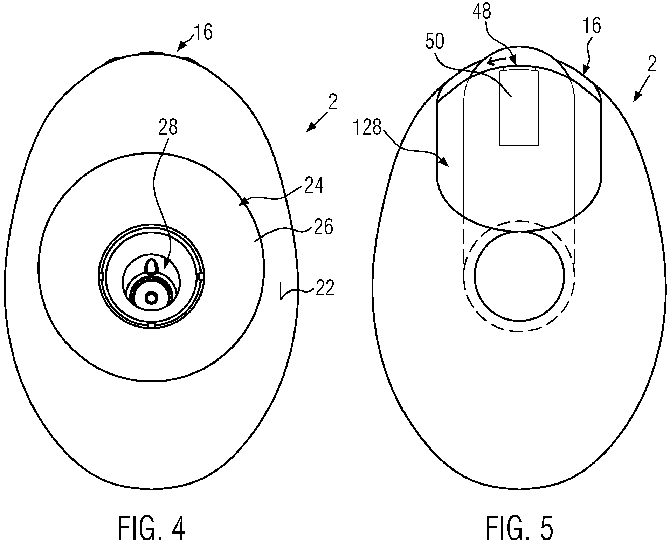

In Fig. 1, reference numeral 2 identifies a housing including a suction source 4 (compare Fig. 2). The housing 2 is made of opaque plastic material. In a side view, the housing is wedge-shaped. A bottom section 6 of the housing 2 is considerably thicker than the top section 8 of the housing 2. The largest width of the housing 2 is essentially level with a longitudinal axis L, which longitudinal axis defines an opening 10 (compare Fig. 4). The housing 2 circumferentially encloses this opening 10. Accordingly, the housing 2 is ring-shaped.

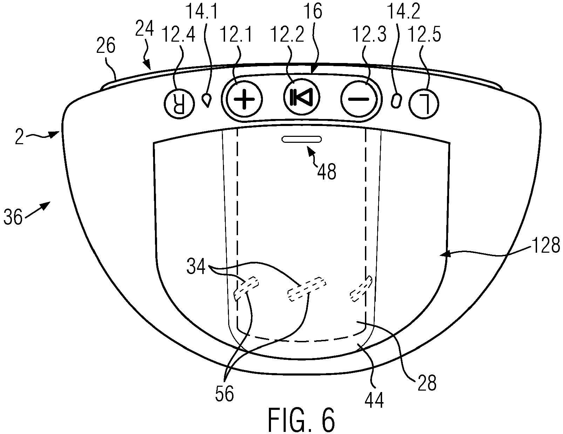

The circumference of the top section 8 is provided with elements 12 of a user interface 16 and optical indicators indicating operational information of the pump. Details of the elements 12 and the optical indicators 14 are derivable from Fig. 6.

As derivable from Fig. 1, a front surface 20 of the housing 2 is flat, whereas a rear surface 22 of the housing 2 is concave.

In Fig. 1, reference numeral 24 identifies a breast shield element of a transparent material, which breast shield element 24 has a flange 26 and a nipple tunnel 28. Next to a free inner end 30 of the nipple tunnel 28, Fig. 1 shows a two-way valve element 32 which two-way valve element 32 can be mounted on the free inner end 30 of the nipple tunnel 28.

The breast shield element 24 is provided with threads 34 arranged circumferentially spaced relative to each other on the outer circumference of the nipple tunnel 28.

Reference numeral 36 identifies a milk container consisting of a spherical container shell element 38 and a lid element 40. This lid element 40 comprises a flat lid section 42 and a longish nipple tunnel receptacle 44. Reference numeral 46 identifies a milk chamber defined between the inner wall of the spherical container shell element 38 and the flat lid section 42. Into said milk chamber 46, the nipple tunnel receptacle 44 extends.

A top section of the spherical container shell element 38 is provided with a spout 48. The flat lid section 42 slidingly holds a spout closure element 50, which is adapted to assume three different positions.

The inner circumference of the nipple tunnel receptacle 44 is provided with threads 56 cooperating with the threads 34 of the nipple tunnel 28 to secure the breast shield element 24 against the milk container 36 when the nipple tunnel 28 projects through the opening 10 of the housing 2 and is received within the nipple tunnel receptacle 44.

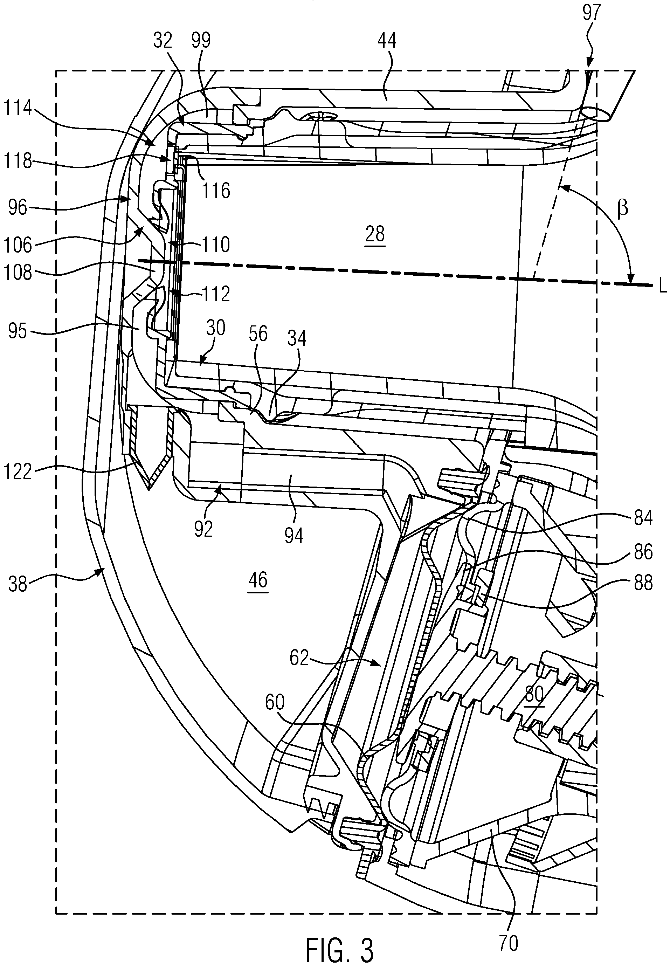

Reference numeral 60 identifies a protective membrane element, which is sandwiched between the milk container 36 and the housing 2 closing a membrane working chamber 62 formed by a trough projecting from the flat lid section 42 toward the milk chamber 46., see Fig. 3

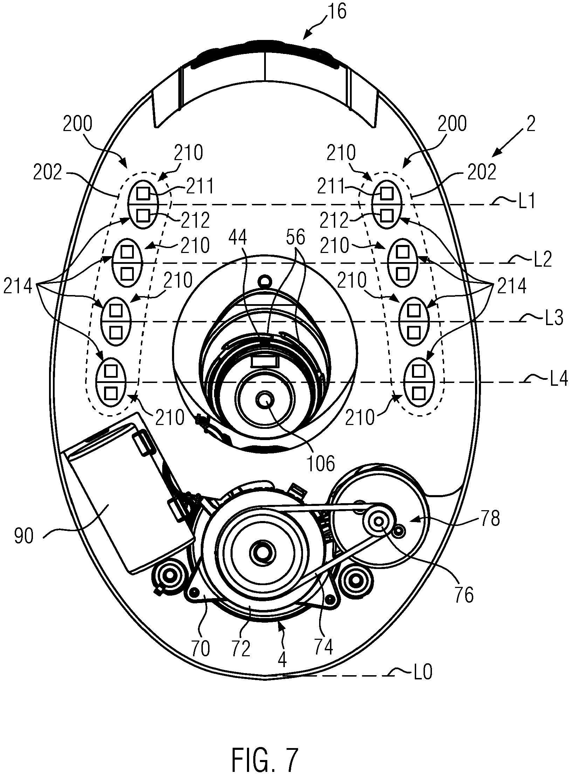

Fig. 3 shows details of the suction source 4 which in combination with Fig. 7 can be understood as comprising a drive housing 70 rotatably supporting a nut element 72 which nut element 72 cooperates with a drive belt 74 to drivingly connect the nut element 72 with a drive shaft 76 of an electric drive motor 78.

The nut element 72 cooperates with a spindle 80. The nut element 72 is rotatably supported by a roller bearing 82 held by the drive housing 70. Between the drive housing 70 and walls of the housing 1, an aggregate membrane 84 is sandwiched, which aggregate membrane 84 is sandwiched between a cap section 86 of the spindle 80 and a disc element 88. This disc element 88 can be secured against the cap section 86, e. g. by means of gluing, welding or hot pressing of projections which penetrate the aggregate membrane and the disc element 88 and the like.

Reference numeral 90 identifies a rechargeable battery/accumulator adapted to energize the electric drive motor 78 and being electrically coupled with a USB interface below the cover 18 for recharging the battery 90, see Fig. 7.

In particular, Fig. 3 elucidates details of a pumping chamber 92 including the membrane working chamber 62, a pumping channel 94, an outlet space 95 provided between the free inner end 30 of the nipple tunnel 28 and a forward closed end 96 of the nipple tunnel receptacle 44 and an annular milk path 99. As derivable from Fig. 3, the front surface 20 of the housing 2 is inclined relative to the longitudinal axis L by an angle β of about 80°. A gap 97 is provided between the milk container 36 and the housing 2 extending with this angle of inclination β relative to longitudinal axis L of the nipple tunnel. In Fig. 2, the gap 97 has a very small thickness. The gap 97 may be larger to receive textile material of a bra (not shown).

The closed end 96 has a projection 106 defining a flat closing surface 108 cooperating with the two-way valve element 32. The center of the two-way valve element 32 has a reflux opening 110, which in cooperation with the flat closing surface 108 defines a reflux valve 112.

In Fig. 3, reference numeral 114 identifies a milk outlet opening cooperating with a wall section 116 projecting radially inwardly from the nipple tunnel 26 and provided by the breast shield element 38. This wall section 116 in combination with the milk outlet opening 114 defines a milk outlet valve 118.

As derivable from Fig. 3, the lower end of the outlet space 95 is provided with a container valve 122 sealing the pumping chamber 92 against the milk chamber 46. This container valve 122 prevents reflux of milk contained within the milk chamber 46 into the outlet space 95. The container valve 122 is formed by a separate element formed made of a soft elastomeric material and attached to the milk container 36, specifically, the nipple tunnel receptacle 44. Preferably, the container valve 122 is assembled when connecting a closure element to the nipple tunnel receptacle 44. Thus, the container valve 122 may be affixed to the lid element 40 or may be separatable from the lid element 40 for cleaning or replacement.

For operation, the nipple will be arranged within the nipple tunnel 28. Proper alignment can be observed through the window 128 from above. Next, the pump is activated. Thus, a negative pressure is built up in the pumping chamber 92. The reflux valve 112 is closed. The negative pressure will build up in the nipple tunnel 28 through the open milk outlet valve 118. Extracted milk is drawn through the milk outlet opening 114 in a reduction cycle of the suction source 4. In an expansion cycle, in which the aggregate membrane 84 and thus the protective membrane element 60 is moved towards the membrane working chamber 62, the milk outlet valve 118 will close. The suction pressure within the pumping chamber 92 will be reduced, i.e. the pressure will rise. Until a reduced threshold suction pressure is reached within the nipple tunnel 28, the reflux valve 112 will remain open. Thus, a negative pressure will be maintained as a minimum suction pressure within the nipple tunnel 28. At appropriate pressure difference, milk M will flow through the container valve 122, which is a one-way valve, into the milk chamber 46. In the reduction cycle, in which the aggregate membrane 84 and thus the protective membrane element 60 is moved towards the drive housing 70, the suction force within the pumping chamber 94 is increased. Eventually, the container valve 122 as a one-way valve will close to prevent reflux of milk M from the milk chamber 46 into the pumping chamber 92. The milk outlet valve 118 will open to allow milk to flow from the nipple tunnel 28 into the outlet space 95 of the pumping chamber, which pumping chamber 92 is essentially completely filled with milk M. Thus, the level of extracted milk M within the milk chamber 46 builds up during operation of the pump while the nipple tunnel 28 is filled with milk M and the nipple is constantly provided within a negative pressure environment.

Fig. 1 shows the breast pump 1 shaped at least in part to fit inside a bra and which is built to accumulate milk M expressed from a breast of a lactating mother in a milk container 36. The breast pump 1 comprises the housing 2, the suction source 4, the breast shield element 24 with the nipple tunnel 28 adapted to receive a nipple and the milk container 36 adapter to contain a predetermined amount of milk M.

As can be seen in Fig. 1 and 7, the breast pump 1 further comprises eight sensor means 210 each including an emitter 211 for an emission of electromagnetic waves and a receiver 212 for a reception of the electromagnetic waves, each sensor means 210 designed to provide a sensor signal for a detection of milk M. The electromagnetic waves are in the form of infrared light.

The breast pump 1 further comprises a controller 200 with a printed circuit board 202 for controlling the suction source 4 and adapted to receive the sensor signals.

The milk container 36 further includes eight transparent sections 360 each of which is at least partially transparent to the electromagnetic waves and is arranged to be in contact with the milk M.

The transparent sections 360 as well as the sensor means 210 are located pairwise on one of four fill levels L1, L2, L3, L4. On the flat lid section 42 there are scale lines 130 which correspond the four fill levels L1, L2, L3, L4. The highest fill level L1, L2, L3, L4 which is the first fill level L1 pertains to the amount of 120 ml milk in the milk container 36, especially when it is in an upright position.

The transparent sections 360 are made monolithically with the lid element 40, in particular with the flat lid section 42 in order to have a flush transition between the crystal clear, smooth and scratch resistant surface of the transparent sections 360 to the rather opaque surface of the rest of the flat lid section 42.

As can be seen in Fig. 1 and 7, the eight sensor means 210 are arranged to each face one of the transparent section 360 at one of four fill levels L1, L2, L3, L4, especially in order to emit electromagnetic waves towards a direction and receive the electromagnetic waves from said direction, where four pairs of two sensor means 210 correspond to one of the four fill levels L1, L2, L3, L4. The lowermost fill level L0 corresponding to an empty milk container 36 is essentially placed in the region of the hinge 52.

The fill levels L1, L2, L3, L4 each correspond to a certain amount of milk M in the milk container 36 when the milk container 36 is in an upright position (cf. Fig. 7). However, in case the milk container 36 is somewhat tilted relative to the upright position, the fill levels L1, L2, L3, L4 - when reached by the milk M - correspond to different amounts of milk M in the milk chamber 46 relative to when the milk container 36 is in the upright position. It may even be that not both the two sensor means 210 of one fill level L1, L2, L3, L4 detect the presence of milk M simultaneously. As such, however, the four pairs of sensors 210 each on one of the fill levels L1, L2, L3, L4 make possible an accurate measurement of the actual amount of milk in the milk container somewhat independent from the upright position of the milk container 36 and/or the angle the milk container 36 is tilted. This is because from frequent tilting during a mother's use the sensor means 210 frequently may detect presence of milk M and then absence of milk M. When it is considered that within a period of e.g. up to 10 seconds the actual amount of milk M in the milk container 36 may essentially not even change during expression by a significant amount, the switch within two or more sensor signals between presence and absence of milk M may make possible to derive the actual amount of milk M. For example, it may have been evaluated in experiments how the sensor signals look like over time (e.g. the 10 seconds) upon tilting the breast pump 1, and the experiments may have been conducted for a variety of specific amounts of milk M filled into the milk container 36. The controller 200 may calculate the actual amount of milk M under consideration of all eight sensor signals.

The opening 10 of the housing 2 for the nipple tunnel 28 is arranged below the first fill level L1 and on all fill levels L1, L2, L3, L4 between the two sensor means 210.

The transparent sections 360 are each monolithically integrated in the milk container 36 in that the lid element 40 with the transparent sections 360 is made as a one-piece injection molded part from amorphous plastics. The transparent sections 360 are each provided with a smooth and crystal clear transparent material/surface, e.g. with an Ra < 0.1 micrometers, as opposed to the surrounding which is rather opaque.

The controller 200 is adapted to receive sensor signals, especially the sensor signal of all of the sensor means 210, 230, 240. In this regard, the printed circuit board 202 may hold mounted a CPU, GPU, RAM, storage, and in particular holds the sensor means 210, 230.

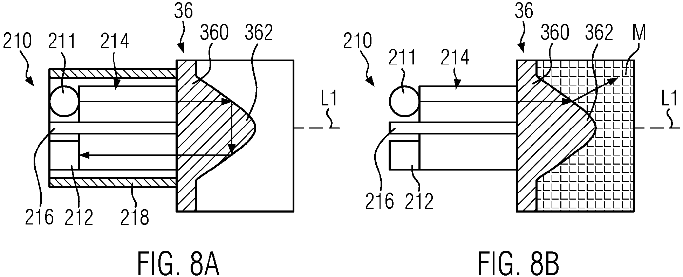

Particularly illustrated in Fig. 8A-B is a schematic section of the arrangement of the sensor means 210 relative to the transparent section 360 of the milk container 36 in an assembled state of the breast pump 1 of Fig. 1-7 and at the first fill level L1. As can be seen, each of the sensor means 210 is in contact with a light guidance element 214 in the form of a lens that is held in the housing 8. The emitter 211 and the receiver 212 are divided by a crosstalk inhibitor 216 that inhibits crosstalk. The crosstalk inhibitor 216 divides the light guidance element as well. Atop the light guidance element 214 and opposite the sensor means 210 there may be an encapsulation element 218 in the form of a plastic surface layer protecting the light guidance element 214. The light guidance element 214 is facing the and is in contact with the milk container 36.

As a modification of the transparent section 360 as shown in Fig. 9A-B, it is shown in the Fig. 10A-B that as shown in Fig. 8A-B, the transparent section 360 has a protrusion 362 affecting the path of the infrared electromagnetic waves from the emitter 211 to the receiver 212. The protrusion 362 is cone shaped, V-shaped and/or roof-shaped and the cone shape faces towards a direction opposite the sensor means 210 and/or towards the milk chamber 46 in the milk container 36. Both the emitter 211 relative to receiver 212 and the light guidance element 214 itself are divided by one crosstalk inhibitor 216.

In the absence of milk M it is indicated in Fig. 8A by a straight arrow from the emitter 211 and two subsequent straight arrows that the emitted infrared electromagnetic waves pass through the light guidance element 214 and are reflected twice within the transparent section 360 and thus backwards towards the receiver 212.

In the presence of milk M it is indicated in Fig. 8B that the emitted infrared electromagnetic waves are refracted within the protrusion 362 and directed through the milk M and/or the milk chamber 46.

Here, the protrusion 362 enables that the electromagnetic waves are only reflected back to the sensor means 210 in case the (empty) milk container 36 is correctly attached. This provides a possibility to check a correct attachment of the milk container 36 by considering the sensor signal upon a start of the pumping with the breast pump 1 with the empty milk container 36.

### List of Reference Signs

- 1: breast pump
- 2: housing
- 4: suction source
- 6: bottom section
- 8: top section
- 10: opening
- 12: element of a user interface
- 14: optical indicator
- 16: user interface
- 18: cover
- 20: front surface
- 22: rear surface
- 24: breast shield element
- 26: flange of the breast shield element
- 28: nipple tunnel
- 30: free inner end
- 32: two-way valve element
- 34: thread
- 36: milk container
- 38: container shell element
- 40: lid element
- 42: lid section
- 44: nipple tunnel receptacle
- 46: milk chamber
- 48: spout
- 50: spout closure element
- 52: hinge
- 56: thread
- 60: protective membrane element
- 62: membrane working chamber
- 70: drive housing
- 72: nut element
- 74: drive belt
- 76: drive shaft
- 78: drive motor
- 80: spindle
- 82: roller bearing
- 84: aggregate membrane
- 86: cap section
- 88: disc element
- 90: battery
- 92: pumping chamber
- 94: pumping channel
- 95: outlet space
- 96: closed end
- 97: gap
- 99: annular milk path
- 106: projection
- 108: flat closing surface
- 110: reflux opening
- 112: reflux valve
- 114: milk outlet opening
- 116: wall section
- 118: milk outlet valve
- 122: container valve

- 128: window
- 130: scale line

- L: longitudinal axis
- β: angle between front surface 20 of the housing 2 and longitudinal axis L

- 200: controller
- 202: printed circuit board
- 210: sensor means
- 211: emitter
- 212: receiver
- 214: light guidance element
- 216: crosstalk inhibitor
- 218: encapsulation element
- 360: transparent section
- 362: protrusion
- L0: lowermost level
- L1: first fill level
- L2: second fill level
- L3: third fill level
- L4: fourth fill level

## Claims

1. A breast pump (1) shaped at least in part to fit inside a bra and built to accumulate milk (M) expressed from a breast of a lactating mother in a milk container (36), the breast pump (1) comprising
- a housing (2),
- a suction source (4),
- a breast shield element (24) with a nipple tunnel (28) adapted to receive a nipple,
- a sensor means (210) including an emitter (211) for an emission of electromagnetic waves and a receiver (212) for a reception of the electromagnetic waves, designed to provide a sensor signal for a detection of the milk (M),
- a controller (200) for controlling the suction source (4) and adapted to receive the sensor signal, and
- a milk container (36) adapted to contain a predetermined amount of expressed milk (M) and including a transparent section (360) which is at least partially transparent to the electromagnetic waves and arranged to be in contact with the milk (M),
**characterized in that**
the transparent section (360) has a protrusion (362) arranged to be in front of and to face away from the corresponding sensor means (210) in order to be wetted by the milk (M) and in order to reflect and/or refract the electromagnetic waves from the emitter (211).

2. The breast pump (1) according to any one of the previous claims, **characterized in that** an opening (10) of the housing (2) for the nipple tunnel (28) is arranged
- below the first fill level (L1), and/or
- above the fill level (L1), and/or
- between two of the sensor means (210).

3. The breast pump (1) according to any one of the previous claims, **characterized in that** a spout (48) of the milk container (36) adapted to pour milk (M) out of the milk container (36) and/or
a venting hole of the milk container (36) for a venting of the milk container (36) are/is arranged
- above the first fill level (L1), and/or
- between two of the sensor means (210).

4. The breast pump (1) according to any one of the previous claims, **characterized by** the protrusion (362) and/or the transparent section (360) having at least one of a prism, a nose, a cone, a V-shape, a roof-shape, a spherical segment and a pyramid.

5. The breast pump (1) according to any one of the previous claims, **characterized in that** the transparent section (360) is monolithically integrated in the milk container (36).

6. The breast pump (1) according to any one of the previous claims, **characterized in that** the transparent section (360)
- is harder and/or more transparent to the electromagnetic waves relative to a different section of the milk container (36) and/or
- is made of plastics, in particular amorphous plastics.

7. The breast pump (1) according to any one of the previous claims, **characterized by** a light guidance element (214), such as a lens and/or a light pipe, in particular wherein at least one or all of the sensor means (210) is/are in contact with the light guidance element (214).

8. The breast pump (1) according to any one of the previous claims, **characterized in that** a crosstalk inhibitor (216) is provided, especially dividing the light guidance element (214).

9. The breast pump (1) according to any one of the previous claims, **characterized by** an encapsulation element (218), in particular wherein the light guidance element (214) is covered by the encapsulation element (218).

10. The breast pump (1) according to claim 7, 8 or 9 **characterized in that** on the side opposite the sensor means (210) the light guidance element (214) and/or the encapsulation element (218) are/is arranged at a distance to the transparent section (360) of no more than 1 mm, especially is in contact therewith.

11. The breast pump (1) according to any one of the previous claims, **characterized in that** the housing (2), the suction source (4), the breast shield element (24), the sensor means (210) and the milk container (36) are shaped to be joined for forming a breast pump (1) adapted to fit inside the bra.

12. The breast pump (1) according to any one of the previous claims, **characterized in that** the suction source (4), the breast shield element (24), the sensor means (210) and the milk container (36) are supported by and/or attached to the housing (2).
